# EUROPÄISCHE PATENTSCHRIFT

(11) **EP 0 985 668 B1**
(45) Veröffentlichungstag und Bekanntmachung des Hinweises auf die Patenterteilung: **07.11.2001**
(21) Anmeldenummer: 99115470.9
(22) Anmeldetag: 05.08.1999
(51) Int. Cl.: C07D 233/90

(54) **Verfahren zur Herstellung von 1,3-Dimethyl-imidazolium-4-carboxylat**
Process for preparation of 1,3-dimethyl-imidazolium-4-carboxylate
Procédé pour la préparation de 1,3-diméthyl-imidazolium-4-carboxylate

(30) Priorität: 12.08.1998 DE 19836477
(43) Veröffentlichungstag der Anmeldung: 15.03.2000
(73) Patentinhaber: BASF AKTIENGESELLSCHAFT, 67056 Ludwigshafen (DE)
(72) Erfinder: Fischer, Jakob, Dr., 85414 Kirchdorf (DE); Siegel, Wolfgang, Dr., 67117 Limburgerhof (DE); Bomm,Volker, Dr., 67112 Mutterstadt (DE); Fischer, Martin, Dr., 67071 Ludwigshafen (DE); Mundinger, Klaus, Dr., 67117 Limburgerhof (DE)

(56) Entgegenhaltungen:
- EP-A- 0 291 074
- US-A- 5 856 513
- M. LISSEL: LIEBIGS ANNALEN DER CHEMIE, 1987, Seiten 77-9, XP002125053
- M. LISSEL ET AL.: SYNTHESIS, Mai 1986 (1986-05), Seiten 382-3, XP002125054
- PATENT ABSTRACTS OF JAPAN vol. 1998, no. 5, 30. April 1998 (1998-04-30) & JP 10 017553 A (MITSUBISHI CHEM CORP), 20. Januar 1998 (1998-01-20)
- A. J. WEINHEIMER ET AL.: TETRAHEDRON, Bd. 29, Nr. 20, 1973, Seiten 3135-6, XP002125055
- T. JAHN ET AL.: TETRAHEDRON LETTERS, Bd. 38, Nr. 22, 1997, Seiten 3883-4, XP004064871

## Beschreibung

Die vorliegende Erfindung betrifft ein Verfahren zur Herstellung von 1,3-Dimethyl-imidazolium-4-carboxylat.

1,3-Dimethyl-imidazolium-4-carboxylat findet gemäß der zeitgleichen deutschen Anmeldung Nr. 19836474.1 Anwendung als Katalysator bei der Herstellung von Isophoronnitril (IPN) aus Isophoron und Cyanwasserstoff.

1,3-Dimethyl-imidazolium-4-carboxylat, ein Betain der Formel I 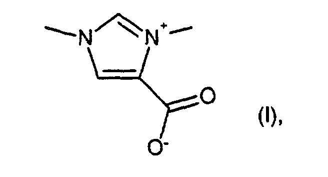 ist als Naturstoff unter dem Namen Norzooanemonin bekannt, dessen Isolierung und Charakterisierung in Tetrahedron Lett. 38, 3883-4 (1997) beschrieben wurde.

In Tetrahedron 29, 3135-6 (1973) wird 1,3-Dimethyl-imidazolium-4-carboxylat durch Umsetzung von Imidazol-4-carbonsäure mit Dimethylsulfat dargestellt. Nachteilig an diesem Verfahren ist der notwendige Umgang mit dem hoch toxischen Dimethylsulfat.

Der vorliegenden Erfindung lag die Aufgabe zugrunde, ein alternatives wirtschaftliches Verfahren zur Herstellung von 1,3-Dimethyl-imidazolium-4-carboxylat aufzufinden.

Demgemäß wurde gefunden, daß man 1,3-Dimethyl-imidazolium-4-carboxylat durch Umsetzung von 1-Methyl-imidazol mit Dimethylcarbonat herstellen kann.

Aus der EP-A-291 074 ist bekannt, daß Dimethylcarbonat bei dessen Umsetzung mit einer etwa stöchiometrischen Menge eines tertiären Amins als Methylierungsmittel fungiert. So liefert beispielsweise die Umsetzung von Triethylamin mit Dimethylcarbonat das Produkt Triethylmethylammonium-methylcarbonat in 89,9 % Ausbeute (loc. cit., Beispiel 1).

In Synthesis, 382-3 (1986) und Liebigs Ann. Chem., 77-9 (1987) wird die Synthese von 1-Methylimidazol in 91 % Ausbeute durch Umsetzung von Imidazol mit Dimethylcarbonat in Gegenwart eines Katalysators beschrieben. Die fehlenden 9 % Ausbeute sind laut Liebigs Ann. Chem. (1987), Seite 77, 2. Spalte, 4. Absatz, auf unumgesetztes Imidazol zurückzuführen. Diese Publikation lehrt ebenfalls, daß ein Angriff des Imidazols bzw. des 1-Methyl-imidazols auf die Carbonylgruppe des Dimethylcarbonats nicht stattfindet.

Weiterhin ist aus JP-A-10 17,553 (Chem. Abstracts 128: 167423p) und aus JP-A-10 17,554 (Chem. Abstracts 128: 167424q) bekannt, daß N-Alkylimidazoline durch Umsetzung mit Dimethylcarbonat am Stickstoffatom in der 3-Position methyliert werden.
So liefert die Reaktion von 1-Ethyl-2-methyl-imidazolin mit (MeO)₂CO das Produkt 1-Ethyl-2,3-dimethyl-imidazolinium-methylcarbonat in 98 % Ausbeute.

Im Lichte der oben zitierten Dokumente war zu erwarten, daß die Umsetzung von 1-Methyl-imidazol mit Dimethylcarbonat nach folgender Gleichung das Produkt 1,3-Dimethyl-imidazolium-methylcarbonat liefert.

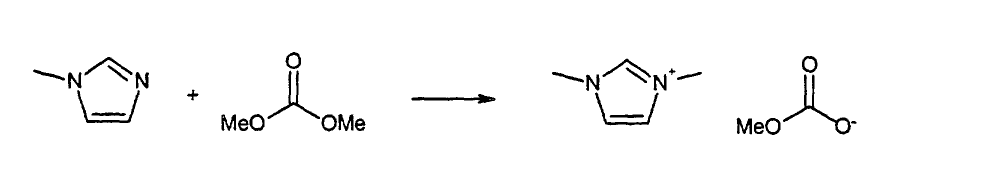

Überraschenderweise wurde jedoch gefunden, daß die Umsetzung von 1-Methyl-imidazol mit Dimethylcarbonat in hohen Ausbeuten zum 1,3-Dimethyl-imidazolium-4-carboxylat der Formel I führt:

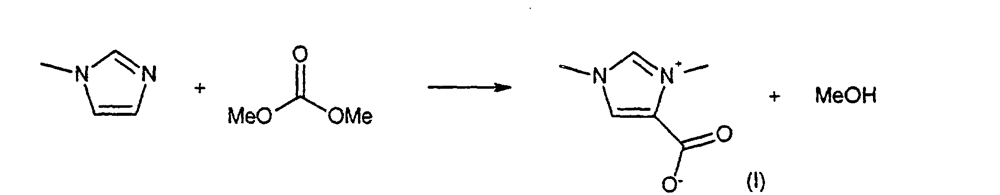

Das erfindungsgemäße Verfahren läßt sich wie folgt durchführen.

Im allgemeinen wird 1-Methyl-imidazol zusammen mit 0,1 bis 2 Moläquivalenten, bevorzugt 0,5 bis 1,5 Moläquivalenten, besonders bevorzugt 0,9 bis 1,1 Moläquivalenten, ganz besonders bevorzugt einem Moläquivalent, Dimethylcarbonat, gegebenenfalls zusammen mit einem Lösungsmittel, vorgelegt und die Mischung anschließend unter Rühren auf Temperaturen von 50 bis 200 °C, bevorzugt 100 bis 180 °C, erhitzt.

In einer bevorzugten Ausführungsform wird das 1-Methyl-imidazol, gegebenenfalls zusammen mit einem Lösungsmittel, im Reaktionsgefäß vorgelegt, auf eine Temperatur von 50 bis 200 °C, bevorzugt 100 bis 180 °C, erhitzt und anschließend 0,5 bis 1,5 Moläquivalente, bevorzugt 0,9 bis 1,1 Moläquivalente, besonders bevorzugt ein Moläquivalent, Dimethylcarbonat zugefahren.

Als Lösungsmittel eigen sich z.B. aliphatische oder aromatische Lösungsmittel, wie Pentan, Hexan, Benzol, Toluol, Xylol, Ether, wie Methyl-tert.-butylether, Diethylether, Tetrahydrofuran, Dioxan, Amide, wie Dimethylformamid, Dimethylacetamid, N-Methylpyrrolidon, Harnstoffe, wie N,N'-Dimethylethylenharnstoff, N,N'-Dimethylpropylenharnstoff und N,N,N',N'-Tetra-n-butylharnstoff, oder zusätzliches Dimethylcarbonat.

Besonders bevorzugt wird die Umsetzung in Abwesenheit eines ) Lösungsmittels durchgeführt.

Die Reaktionszeiten sind im allgemeinen von der Reaktionstemperatur abhängig und umso kürzer, je höher die gewählte Reaktionstemperatur ist. Die Reaktionszeiten betragen im allgemeinen 5 Stunden bis 3 Tage, bevorzugt 12 bis 24 Stunden.

Die Umsetzung wird im allgemeinen bei einem Druck (absolut gemessen) von 0,05 bis 5 MPa, bevorzugt 0,1 bis 1 MPa, durchgeführt. Besonders bevorzugt erfolgt die Umsetzung in einem geschlossenen Reaktionsgefäß unter Eigendruck.

Die Umsetzung kann sowohl kontinuierlich als auch diskontinuierlich in gängigen Reaktionsgefäßen bzw. Reaktoren, wie z.B. Rührreaktoren, Rohrreaktoren, Rührbehälterkaskaden, durchgeführt weri den.

Die Reinigung des Umsetzungsprodukts I erfolgt zweckmäßigerweise durch Kristallisation. Geeignete Lösungsmittel sind hierbei Alkohole, wie Methanol, Ethanol, Propanol, Ether, wie Methyl-tert.-butylether, Diethylether, Tetrahydrofuran, Dioxan, Ketone, wie Aceton, Diethylketon, und Ester, wie Essigsäureethylester.

### Beispiel

### Herstellung von 1,3-Dimethyl-imidazolium-4-carboxylat (Norzooanemonin)

In einem Autoklaven wurden 0,9 mol (73,8 g) 1-Methyl-imidazol und 0,9 mol (81,0 g) Dimethylcarbonat bei Raumtemperatur vorgelegt.Der Ansatz wurde anschließend auf 140 °C erwärmt und 20 Stunden bei dieser Temperatur gerührt (Eigendruck: ca. 0,5 MPa). Nach dem Abkühlen auf Raumtemperatur verblieb eine gelbe dicke Suspension. Die Kristalle wurden abfiltriert und im Ölpumpenvakuum getrocknet. Rohgewicht: 119,4 g (= 94,7 % Rohausbeute).

Die Kristalle wurden aus einem ca. 1/1 Ethanol/Methanol-Gemisch umkristallisiert. Dabei fielen weiße Kristalle an. Diese wurden abfiltriert und im Hochvakuum getrocknet. Gewicht: 48,9 g.

Die Mutterlauge wurde zur Trockene eingeengt. Der verbleibende, gelb-ölige Rückstand wurde erneut in heißem 1/1 Ethanol/Methanol-Gemisch aufgenommen und in der Kälte ausgefällt. Es resultierten weitere 53,9 g Produkt.
Gesamtausbeute: 102,8 g (81,5 %).
Charakterisierung des Produkts:
Schmelzpunkt: 240 °C (Zersetzung).
MS (Elektronenspray Ionisation ESI, Direkteinlaß): M = 141 (M+H)⁺.
Elementaranalyse: berechnet: C 51,4; H 5,8; N 20,0; O 22,8
C₆H₈N₂O₂; MG = 140,14 gefunden: C 51,3; H 5,7; N 19,9; O 23,4.
1H-NMR (400 MHz, D₂O) δ (ppm) = 3,98 (3 H); 4,12 (3H); 7,88 (1H); 1 H tauscht in D₂O aus.
13C-NMR (100,61 MHz, D₂O) δ (ppm) = 38,55 (Methyl); 38,65 (Methyl); 129,03 (CH); 133,59 (C-COO-); 140,82 (t, Kopplung C-D); 165,8 (COO-).
IR (KBr), [cm⁻¹] : 3451 ss, 1621 ss.

## Patentansprüche

1. Verfahren zur Herstellung von 1,3-Dimethyl-imidazolium-4-carboxylat, **dadurch gekennzeichnet, daß** man 1-Methylimidazol mit Dimethylcarbonat umsetzt.

2. Verfahren nach Anspruch 1, **dadurch gekennzeichnet, daß** man 1-Methyl-imidazol mit 0,5 bis 1,5 Moläquivalenten Dimethylcarbonat umsetzt.

3. Verfahren nach den Ansprüchen 1 und 2, **dadurch gekennzeichnet, daß** man die Umsetzung bei Temperaturen von 50 bis 200 °C durchführt.

4. Verfahren nach den Ansprüchen 1 bis 3, **dadurch gekennzeichnet, daß** man die Umsetzung in Abwesenheit eines Lösungsmittels durchführt.

## Claims

1. A process for the preparation of 1,3-dimethylimidazolium 4-carboxylate, which comprises reacting 1-methylimidazole with dimethyl carbonate.

2. A process as claimed in claim 1, which comprises reacting 1-methylimidazole with from 0.5 to 1.5 mole equivalents of dimethyl carbonate.

3. A process as claimed in claims 1 and 2, which comprises carrying out the reaction at from 50 to 200°C.

4. A process as claimed in claims 1 to 3, which comprises carrying out the reaction in the absence of a solvent.

## Revendications

1. Procédé de préparation du 1,3-diméthyl-imidazolium-4-carboxylate, **caractérisé en ce qu'**on fait réagir du 1-méthylimidazole avec du carbonate de diméthyle.

2. Procédé selon la revendication 1, **caractérisé en ce qu'**on fait réagir le 1-méthylimidazole avec de 0,5 à 1,5 équivalents en moles de carbonate de diméthyle.

3. Procédé selon les revendications 1 et 2, **caractérisé en ce que** la réaction est mise en oeuvre à des températures de 50 à 200°C.

4. Procédé selon les revendications 1 à 3, **caractérisé en ce qu'**on met en oeuvre la réaction en l'absence de solvant.
